# EUROPÄISCHE PATENTSCHRIFT

(11) **EP 3 897 443 B1**
(45) Veröffentlichungstag und Bekanntmachung des Hinweises auf die Patenterteilung: **05.08.2026**
(21) Anmeldenummer: 19835654.5
(22) Anmeldetag: 20.12.2019
(51) Int. Cl.: A61B 90/70, B08B 9/043, B08B 9/049

(54) **SPÜLWERKZEUG FÜR EIN HANDSTÜCK EINES CHIRURGISCHEN INSTRUMENTS**
FLUSHING TOOL FOR A HANDPIECE OF A SURGICAL INSTRUMENT
OUTIL DE RINÇAGE POUR PIÈCE À MAIN D'UN INSTRUMENT CHIRURGICAL

(30) Priorität: 21.12.2018 DE 102018133503
(43) Veröffentlichungstag der Anmeldung: 27.10.2021
(73) Patentinhaber: Aesculap AG, 78532 Tuttlingen (DE)
(72) Erfinder: BÜRK, André, 78056 Villingen-Schwenningen (DE); ROTH, Manuel, 78166 Donaueschingen (DE)
(74) Vertreter: Winter, Brandl - Partnerschaft mbB
(86) Internationale Anmeldenummer: PCT/EP2019/086531
(87) Internationale Veröffentlichungsnummer: WO 2020/127874

(56) Entgegenhaltungen:
- EP-A1- 2 105 106
- EP-A1- 3 023 077
- DE-A1- 102010 008 745
- US-A- 4 677 997
- US-A1- 2014 090 194

## Beschreibung

Die Erfindung betrifft eine Spülvorrichtung zur Innenreinigung eines Handstücks eines chirurgischen Instruments, insbesondere eine Spülvorrichtung zur Reinigung der distalen Kugellager im Innenraum eines Handstücks eines chirurgischen Instruments, und ein Spülsystem mit der Spülvorrichtung und dem Handstück.

### Hintergrund der Erfindung

Während der Verwendung von chirurgischen Instrumenten kommt das distale Ende des Instruments mit organischen und anorganischen Substanzen in Kontakt, die sich in Form von Ab- bzw. Anlagerungen am und im Instrument festsetzen. Der Einsatz von wiederverwendbaren chirurgischen Instrumenten, beispielsweise Sägen oder Bohrer, erfordert deshalb vor und/oder nach jeder Benutzung eine sachgemäße Aufbereitung, insbesondere Reinigung und/oder Desinfektion, bei der die im und am Instrument anhaftenden Verschmutzungen wieder entfernt werden.

Dazu werden mehrteilige chirurgische Instrumente, die häufig ein elektrisch betriebenes Handstück und einen in das Handstück einkoppelbaren Werkzeugschaft aufweisen, entkoppelt und separat voneinander gereinigt/aufbereitet. Insbesondere die Reinigung der Lumen oder Hohlkörper des Handstücks mit seinen innenliegenden Flächen und Teilen stellt eine besondere Herausforderung dar. Zu diesem Zweck wird das Handstück bzw. der Hohlkörper des Handstücks mit einer Reinigungslösung durchspült. Die Innendurchspülung mit dem Reinigungsfluid soll Schmutzpartikel, die sich an den innenliegenden Teilen des Hohlkörpers angelagert haben, lösen und aus dem Hohlkörper bzw. Handstück austragen.

### Stand der Technik

Besonders die Reinigung der Zwischenräume im Hohlkörper des Handstücks, insbesondere seiner distalen Kugellager, hat sich als schwierig erwiesen. Aufgrund des geringen Widerstands strömt das Reinigungsfluid beim Durchströmen des Handstücks fast ausschließlich durch die Innenringe bzw. entlang der Innenflächen der Innenringe der distalen Kugellager, sodass die Kugeln, Käfige und Zwischenräume dieser Kugellager einer geringeren Reinigungswirkung unterliegen.

Diese Problematik wird im Stand der Technik bereits aufgegriffen. In der Patentschrift US 6 694 991 B1 wird eine Kugellagerreinigungsvorrichtung offenbart, in die ein zu reinigendes Kugellager eingesetzt und dann mit Reinigungslösung gezielt im Zwischenraum des inneren und äußeren Lagerrings umspült wird. Eine solche Kugellagerreinigungsvorrichtung ist jedoch für fest verbaute Kugellager nicht geeignet. Zudem besteht die offenbarte Kugellagerreinigungsvorrichtung aus einer Vielzahl an Teilen, die ein erhöhten Montage- sowie Demontageaufwand verursachen.

Weiterer Stand der Technik ist zudem aus EP 3 023 077 A1, EP 2 105 106 A1, DE 10 2010 008 745 A1, US 4 677 977 A und US 2014 / 0 090 194 A1 bekannt.

Der Stand der Technik hat den Nachteil, dass fest verbaute Kugellager, insbesondere fest im Innenraum / Hohlkörper eines Handstücks eines chirurgischen Instruments verbaute Kugellager, nicht optimal gereinigt werden können.

### Kurze Beschreibung der Erfindung

Die Aufgabe der vorliegenden Erfindung ist es somit, die Nachteile des Stands der Technik zu überwinden oder zumindest zu mildern und insbesondere eine Möglichkeit zur optimalen, zumindest aber verbesserten, Reinigung von Hohlkörpern chirurgischer Instrumente, insbesondere der fest verbauten distalen Kugellager im Handstück (mehrteiliger) chirurgischer Instrumente, zu schaffen.

Die Aufgabe wird gelöst durch eine Spülvorrichtung mit den Merkmalen des Anspruchs 1 und einem Spülsystem mit den Merkmalen des Anspruchs 9. Vorteilhafte Weiterbildungen der Erfindung sind Gegenstände der beigefügten Unteransprüche.

Ein Grundgedanke der Erfindung besteht darin, eine Spülvorrichtung für ein Handstück eines chirurgischen Instruments zu schaffen, mit dem eine Durchströmung durch das Innere des Handstücks so gelenkt / geführt wird, dass möglichst viele, vorzugsweise sämtliche, Zwischenräume im Inneren bzw. Hohlkörper des Handstücks, insbesondere die fest verbauten Kugellager, von der Durchströmung mit einem Reinigungsfluid, insbesondere Reinigungslösung, erfasst werden. Prinzipiell lässt sich dies dadurch erreichen, dass ein Schafteinsatz an der Spülvorrichtung vorgesehen ist, der in eine Werkzeugaufnahme bzw. einen Werkzeugaufnahmeschacht des Handstücks einsetzbar ist und so dimensioniert ist, dass er die Einsetzöffnung lediglich radial innseitig der im Handstück befindlichen Lager/Kugellager flaschenkorkartig verschließt/dichtet und gleichzeitig eine Austrittsöffnung am distalen Ende/Endabschnitt des Handstücks (Austrittsöffnung der Werkzeugaufnahme) ringförmig offen lässt. Auf diese Weise wird beispielsweise proximal in das Handstück einströmendes Reinigungsfluid durch den Spalt zwischen einem Außen- und Innenring des entsprechenden Kugellagers gezwungen. Die axiale Sicherung der Spülvorrichtung in der Werkzeugaufnahme erfolgt dabei entweder über einen in der Werkzeugaufnahme bereits vorgesehenen Kopplungsabschnitt zum Koppeln eines Werkzeugschafts, in den ein spülvorrichtungsseitig vorgesehener Kopplungsabschnitt nach Art eines Werkzeugschaft-Kopplungsabschnitts greift, oder indem der spülvorrichtungsseitig vorgesehene Kopplungsabschnitt einen Lagerring der Kugellager in der Werkzeugaufnahme hintergreift.

Konkret ist eine Spülvorrichtung vorgesehen, insbesondere ein Spülwerkzeug bzw. Spülhilfsmittel, zum Durchspülen eines Handstücks eines chirurgischen Instruments mit oder aus einem schaftartigen Spüleinsatz, der dafür vorgesehen und ausgelegt ist, in eine am Handstück (in Form eines Hohlkörpers) ausgebildete Werkzeugaufnahme eingesetzt zu werden, und der Spüleinsatz (bzw. die Spülvorrichtung) einen proximalen Kopplungsabschnitt zum Koppeln der Spülvorrichtung mit dem Handstück, einen distalen Abschnitt und einen intermediären (teil)zylindrischen Abschnitt hat, der einen größeren Außenradius als der distale Abschnitt hat, wodurch sich an einem Übergang vom distalen Abschnitt zum intermediären Abschnitt ein umlaufender (also sich in Umgangsrichtung über den gesamtem Umfang erstreckender) Schaftabsatz oder eine umlaufende radiale Stufe ausbildet. Man kann auch sagen, dass am Übergang vom distalen Abschnitt zum intermediären Abschnitt ein umlaufender Vorsprung vorgesehen ist bzw. der intermediäre Abschnitt einen umlaufenden Vorsprung bildet. Dabei ist der distale Abschnitt vorzugsweise (im Wesentlichen) koaxial an den intermediären Abschnitt angesetzt oder einstückig an diesen angeformt.

Unter dem intermediären Abschnitt ist ein Abschnitt zu verstehen, der zwischen dem proximalen Abschnitt und dem distalen Abschnitt angeordnet ist.

Durch die vorstehend beschriebene Geometrie der Spülvorrichtung ist es möglich, die Durchströmung eines Handstücks, nach Einsetzen der Spülvorrichtung in den Hohlkörper / die Werkzeugaufnahme des Handstücks, gezielt zu beeinflussen und so zu lenken, dass bestimmte Strömungspfade verschlossen und andere bestimme Strömungspfade gezielt eingeleitet werden.

Insbesondere ist der Übergang bzw. die Kante vom distalen Abschnitt zum intermediären Abschnitt (aus)gerundet und verleiht der Spülvorrichtung somit vorteilhafterweise zum einen eine erhöhte Festigkeit und verhindert zum anderen eine turbulente Strömungsbildung.

Vorzugsweise ist die Spülvorrichtung integral stoffeinstückig gebildet und weist dadurch vorteilhafterweise eine erhöhte Festigkeit auf.

Bevorzugt weist der distale Abschnitt, vorzugsweise an seinem distalen Ende oder im Bereich seines distalen Endes, ein Turbinenrad bzw. einen Schaufelkranz auf. Dabei handelt es sich bei den Schaufeln insbesondere um schräg zur Längserstreckung des distalen Abschnitts verlaufende Laufschaufeln, die vorzugsweise integral stoffeinstückig mit der Spülvorrichtung ausbildet sind.

Durch das Turbinenrad kann die Spülvorrichtung selbst beim Durchströmen mit einem Fluid um die eigene Längsachse gedreht werden und weiter zur verbesserten Reinigung des Hohlkörpers des Handstücks beitragen.

Vorzugsweise weist die Spülvorrichtung am proximalen Kopplungsabschnitt mindestens zwei (feder)elastisch deformierbare und/oder sich in Längsrichtung der Spülvorrichtung erstreckende Klemmarme bzw. Spreizarme auf. Dabei ist insbesondere vorgesehen, dass der proximale Kopplungsabschnitt der Spülvorrichtung von ihrem proximalen Ende her entlang ihrer Längsrichtung eine (in ihrer Querrichtung) durch den proximalen Kopplungsabschnitt hindurchgehende Ausnehmung aufweist, die den proximalen Kopplungsabschnitt entlang der Längsrichtung der Spülvorrichtung in mindestens zwei elastisch verformbare Klemmarme teilt.

Dabei kann die Ausnehmung im Querschnitt bzw. in frontaler Ansicht des Kopplungsabschnitts je nach Anzahl der vorgesehenen Klemmarme (linear) schlitzförmig, Y-förmig oder sternförmig sein. Auch kann die Ausnehmung sich (über ihren Längsverlauf hin) in Querrichtung der Spülvorrichtung zu deren proximalen Ende hin verbreitern, sodass zum proximalen Ende hin eine elastische Verformung der Klemmarme radial nach innen mehr Spielraum bekommt. Die Klemmarme können auf diese Weise im eingesetzten Zustand der Spülvorrichtung in die Werkzeugaufnahme des Handstücks beispielsweise gegen einen Innenring eines Kugellagers vorgespannt werden/sein und eine reibschlüssige Verbindung mit dem Innenring bilden.

Insbesondere weisen die Klemmarme jeweils einen (in Bezug auf den gesamten Kopplungsabschnitt) radial vorspringenden Rastabschnitt mit einer Abgleitfläche auf, die sich auf das proximale Ende des Klemmarms hin erstreckt. Die Rastabschnitte der Klemmarme können auf diese Art einen radial nach außen vorspringenden Schaftabsatz am proximalen Kopplungsabschnitt bilden. Dabei können die Vorsprünge und/oder die Abgleitflächen an den Klemmarmen sich jeweils über den gesamten äußeren Umfang des Klemmarms erstrecken und/oder können die Abgleitflächen jeweils eine gekrümmte, insbesondere teil-kugelförmige oder teil-ellipsoidförmige, Oberfläche aufweisen oder in Form einer solchen gebildet sein.

Unter dem äußeren Umfang der Klemmarme ist dabei der Umfangsabschnitt zu verstehen, der einen Teil des Umfangs des (im Wesentlichen) zylindrischen proximalen Kopplungsabschnitts darstellt.

Vorteilhafterweise kann die Spülvorrichtung somit allein durch Zusammenschieben der Spülvorrichtung und des Handstücks selbstständig in dieses eintauchen und bei Passieren einer Hinterschneidung in der Werkzeugaufnahme, beispielsweise in Einschubrichtung hinter einem Innenring des Kugellagers, einen Formschluss mit der Hinterschneidung bilden, um die Spülvorrichtung und die Werkzeugaufnahme gegen ein Verschieben axial zueinander zu sichern.

Bevorzugt ist der intermediäre Abschnitt ringförmig und/oder hülsenförmig gebildet.

Besonders bevorzugt weist die Spülvorrichtung, vorzugsweise die gesamte Spülvorrichtung, zumindest aber der distale Abschnitt und der intermediäre Abschnitt der Spülvorrichtung, einen Vollzylinder auf oder ist/sind diese ein Vollzylinder.

Dies verleiht der Spülvorrichtung eine erhöhte Formstabilität und vereinfacht die Fertigung der Spülvorrichtung.

Es ist weiter ein Spülsystem vorgesehen zum Durchspülen eines Handstücks eines chirurgischen Instruments mit der vorstehend beschriebenen Spülvorrichtung und einem Handstück eines chirurgischen Instruments, das eine Werkzeugaufnahme in Form eines Hohlkörpers ausbildet, in den die Spülvorrichtung distal einführbar und (partiell) aufnehmbar ist, wobei die Werkzeugaufnahme einen Kopplungsabschnitt zum Koppeln der Spülvorrichtung mit deren Kopplungsabschnitt aufweist und in einem distalen Abschnitt der Werkzeugaufnahme mindestens ein, vorzugsweise zwei oder mehr, Kugellager vorgesehen ist, das einen eine Durchgangöffnung durch das Kugellager bildenden Innenring aufweist. Dabei verschließt die Spülvorrichtung in gekoppeltem Zustand des Spülsystems, in dem die Spülvorrichtung (zumindest abschnittweise) in der Werkzeugaufnahme des Handstücks aufgenommen und eingekoppelt ist, einen Strömungsweg durch das Handstück entlang einer Innenumfangsfläche bzw. Innenumfangsseite des Innenrings / der Durchgangsöffnung (nahezu vollständig) gegen eine Fluiddurchströmung, vorzugsweise indem die Spülvorrichtung an der Innenumfangsfläche anliegt, und gibt einen Strömungsweg distal vom dem zumindest einen Kugellager wieder frei.

Der Strömungspfad durch die Durchgangsöffnung des Innenrings wird somit blockiert und ein Strömungspfad durch die Lagerzwischenräume, insbesondere durch die Zwischenräume zwischen Kugeln und Käfig und/oder Innenring und/oder Außenring, erzwungen bzw. verstärkt, wodurch Schmutzpartikel besser ausgetragen werden und das Lager eine verbesserte Reinigung erfährt.

Bevorzugt ist der Kopplungsabschnitt der Werkzeugaufnahme (in einem proximalen Abschnitt der Werkzeugaufnahme angeordnet und) dazu angepasst, mit dem Kopplungsabschnitt der Spülvorrichtung eine vorzugsweise werkzeuglose, form- und/oder reibschlüssige Kopplung zu bilden. Dabei handelt es sich insbesondere um eine Werkzeugschaftkopplung, die im Betrieb des chirurgischen Instruments dazu dient, einen austauschbaren Werkzeugschaft in das Handstück einzukoppeln und zu halten. Der Kopplungsabschnitt der Spülvorrichtung kann folglich einfach wie der Kopplungsabschnitt diverser austauschbarer Werkzeugschäfte gestaltet werden, um in dem im Handstück bereits vorhandenen Kopplungsabschnitt eingekoppelt und gehalten zu werden. Auf einen weiteren, gesonderten Kopplungsabschnitt im Handstück kann somit verzichtet werden.

Vorzugsweise liegt die Spülvorrichtung (ver)drehfest an der Innenumfangsfläche des Innenrings an und/oder ist (auf andere Weise) (ver)drehfest mit dem Innenring verbunden. Wird die Spülvorrichtung bei der Durchströmung des Handstücks um ihre eigene Längsachse gedreht, beispielsweise indem ein Turbinenrad am distalen Abschnitt der Spülvorrichtung vorgesehen ist, so wird der Innenring des Kugellagers auch gedreht und durch die Bewegung der Kugeln und des Käfigs werden Schmutzteilchen besser ausgetragen und das Lager folglich besser gereinigt.

Insbesondere bevorzugt verläuft der Kopplungsabschnitt der Werkzeugaufnahme entlang der Innenumgangsfläche des Innenrings oder ist durch diese gebildet und ist dazu angepasst, mit dem Kopplungsabschnitt der Spülvorrichtung eine vorzugsweise werkzeuglose, reibschlüssige Klemmkopplung zu bilden. Durch die Klemmkopplung zwischen Innenring und Spülvorrichtung können der Innenring und die Spülvorrichtung folglich sowohl axial, als auch um Umfangsrichtung zueinander gesichert werden.

Dabei können besonders bevorzugt die Klemmarme der Spülvorrichtung mit dem Innenring eine zylindrische Schnappbefestigung bilden.

Insgesamt werden erfindungsgemäß Vorteile dahingehend erzielt, dass die Reinigungswirkung beim Durchspülen des Handstücks verbessert wird, indem eine gezielte Führung des Reinigungsfluids stattfindet und sowohl die Durchflussmenge sowie die Durchflussgeschwindigkeit des Reinigungsfluids durch das Kugellager erhöht wird, infolgedessen es zu einer stärkeren hydromechanischen Wirkung kommt, welche durch die Drehung der Kugeln und des Käfigs noch weiter verstärkt werden kann.

### Kurzbeschreibung der Figuren

Die Erfindung wird nachstehend anhand von bevorzugten Ausführungsbeispielen unter Bezugnahme auf die beigefügte Zeichnung näher beschrieben. Es zeigen:
Fig. 1 eine perspektivische Seitenansicht einer Spülvorrichtung gemäß einer ersten Ausführungsform der Erfindung;
Fig. 2 eine perspektivische Längsschnittansicht eines Handstücks eines chirurgischen Instruments mit eingekoppelter Spülvorrichtung gemäß der ersten Ausführungsform der Erfindung;
Fig. 3a eine Draufsicht auf ein im Handstück des chirurgischen Instruments fest verbautes Kugellager;
Fig. 3b eine perspektivische Ansicht des in Fig. 3a gezeigten Kugellagers;
Fig. 4 eine perspektivische Draufsicht eines chirurgischen Instruments mit eingekoppelter Spülvorrichtung gemäß der ersten Ausführungsform der Erfindung;
Fig. 5 eine perspektivische Seitenansicht einer Spülvorrichtung gemäß einer zweiten Ausführungsform der Erfindung;
Fig. 6 eine perspektivische Frontalansicht der in Fig. 5 gezeigten Spülvorrichtung;
Fig. 7 eine perspektivische Längsschnittansicht des Handstücks des chirurgischen Instruments mit eingekoppelter Spülvorrichtung gemäß der zweiten Ausführungsform der Erfindung in perspektivischer Seitenansicht;
Fig. 8 eine perspektivische Draufsicht des chirurgischen Instruments mit eingekoppelter Spülvorrichtung gemäß der zweiten Ausführungsform der Erfindung.

Die Figuren sind lediglich schematischer Natur und dienen ausschließlich dem Verständnis der Erfindung. Die gleichen Elemente sind mit denselben Bezugszeichen bezeichnet.

### Detaillierte Beschreibung bevorzugter Ausführungsbeispiele

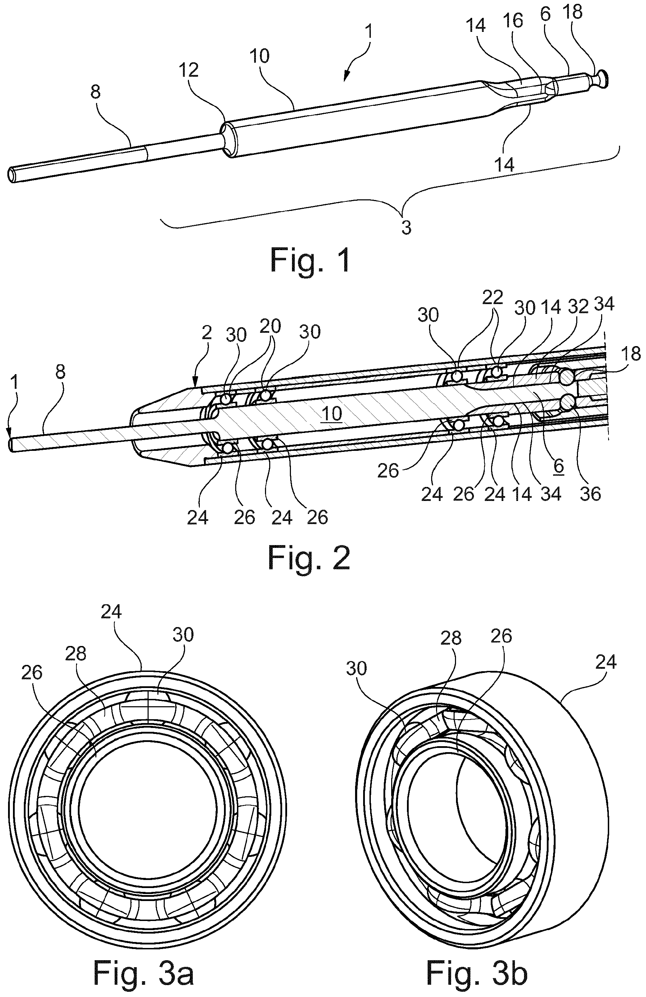

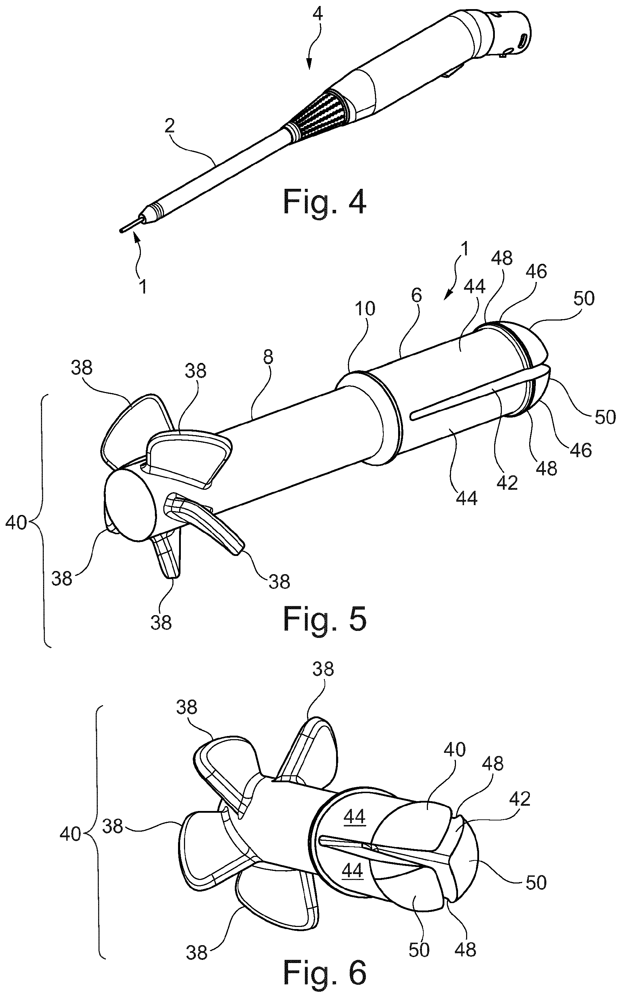

Fig. 1 zeigt eine perspektivische Seitenansicht einer Spülvorrichtung 1 gemäß einer ersten Ausführungsform der Erfindung zum Durchspülen eines Handstücks 2 eines chirurgischen Instruments 4 (zu sehen in Figs. 2 und 4) mit einem schaftartigen Spüleinsatz 3, der dafür vorgesehen und angepasst ist, in eine am Handstück 2 ausgebildete Werkzeugaufnahme eingesetzt zu werden, und die Spülvorrichtung weiter einen proximalen Kopplungsabschnitt 6 zum Koppeln des Spülwerkzeugs 1 mit dem Handstück 2, einen distalen Abschnitt 8 und einen intermediären (teil)zylindrischen Abschnitt 10 hat, der in radialer Richtung der Spülvorrichtung 1 breiter als der distale Abschnitt 8 ausgeführt ist, sodass die Spülvorrichtung 1 an einem Übergang vom distalen Abschnitt 8 zum intermediären Abschnitt 10 eine umlaufende Stufe 12 aufweist. Die Stufe 12 bildet dabei einen gerundeten Übergang vom distalen Abschnitt 8 zum intermediären Abschnitt 10.

Unter dem Spüleinsatz 3 ist insbesondere der Teil der Spülvorrichtung 1 zu verstehen, der im eingesetzten Zustand der Spülvorrichtung 1 in das Handstück 2 in der Werkzeugaufnahme aufgenommen ist.

Der distale Abschnitt 8 ist in Form eines zylindrischen Körpers (runden Querschnitts) gebildet und koaxial zum intermediären Abschnitt 10 angeordnet. Der intermediäre Abschnitt 10 weist einen distalen zylindrischen Teil runden Querschnitts auf und flacht zu seiner proximalen Seite hin ab, sodass der zylindrische Teil (mit gerundetem Übergang) in einen unrunden Teil übergeht, der zwei voneinander abgewandte, im Wesentlichen (plan)parallele, Außenanlageflächen 14 aufweist, welche über zwei konvex gewölbte Seitenflächen 16 miteinander verbunden sind. An diesen unrunden Teil schließt sich der proximale Kupplungsabschnitt 6 an, der ebenfalls im Wesentlichen zylindrisch geformt ist und an seinem proximalen Ende eine gerundete Einkerbung oder Kopplungstaille 18 aufweist.

Die gezeigte Spülvorrichtung 1 ist in Form eines integral stoffeinstückigen, massiven Werkstücks (Vollkörper) gebildet, kann aber selbstverständlich auch durch Fügen mehrere Teile gestaltet werden.

In Fig. 2 ist eine perspektivische Längsschnittansicht eines Handstücks 2 eines chirurgischen Instruments 4 mit eingekoppelter Spülvorrichtung 1 gemäß der in Fig.1 abgebildeten ersten Ausführungsform der Erfindung zu sehen. Das Handstück 2 bildet eine Werkzeugaufnahme in Form eines Hohlkörpers mit einem Innenraum, in den die Spülvorrichtung 1 eingeführt ist. Das Handstück 2 weist zwei distale Kugellager 20 und zwei proximale Kugellager 22 auf, die jeweils einen Außenring 24, einen Innenring 26 und einen zwischen dem Außenring 24 und dem Innenring 26 befindlichen Käfig 28 mit Kugeln 30 haben. Der Aufbau der verbauten Kugellager 20, 22 ist in den Figuren 3a und 3b dargestellt. Da Kugellager aus dem Stand der Technik allgemein bekannt sind, wird auf eine detaillierte Beschreibung der Kugellager 20, 22 verzichtet. In Fig. 2 zu sehen ist weiter, dass die Kugellager 20, 22 über ihre Außenringe 24 fest mit der Außenwand des Handstücks 2 verbunden und in dieses integriert sind. Im proximalen Abschnitt des Handstücks 2 ist eine Führungshülse 32 zu sehen, die einander gegenüberliegende, im Wesentlichen komplementär zu den Außenanlageflächen 14 der Spülvorrichtung 1 gebildete Innenanlageflächen 34 aufweist und die sich durch den Innenring 26 des proximaleren Kugellagers 22 hindurchstreckt und mit diesem in Anlagekontakt steht. Am proximalen Ende der Führungshülse 32 sind Kopplungskugeln 36 vorgesehen.

Im dargestellten eingekoppelten Zustand der Spülvorrichtung 1 in das Handstück 2 greifen die Kopplungskugeln 36 in die Kopplungstaille 18 am proximalen Ende der Spülvorrichtung 1, sodass die Spülvorrichtung 1 und das Handstück 2 axial zueinander gesichert sind. Dabei stehen die im Wesentlichen planen Außenanlageflächen 14 der Spülvorrichtung 1 in Anlage(flächen)kontakt mit den Innenanlageflächen 34 der Führungshülse 32, wodurch die Spülvorrichtung 1 und die Führungshülse 32 drehfest aneinander gehalten sind.

Der Außendurchmesser des intermediären Abschnitts 10 ist dabei so bemessen, dass der zylindrische Teil des intermediären Abschnitts 10 die Durchgangsöffnungen der Innenringe 26 der Kugellager 20 verschließt, beispielsweise indem er mit den Innenumfangsflächen der Innenringe 26 in Anlagekontakt tritt oder zwischen den Innenumfangsflächen der Innenringe 26 und der äußeren Zylinderoberfläche des zylindrischen Teils des intermediären Abschnitts 10 ein Spaltmaß von bevorzugt weniger als 0,1 mm vorliegt, das einen Strömungspfad zwischen den Innenumfangsflächen und der äußeren Zylinderoberfläche stark reduziert, um beim Durchströmen des Handstücks 2 mit einem Reinigungsfluid einen Strömungsweg jeweils durch den Zwischenraum zwischen dem Außenring 24 und dem Innenring 26 der Kugellager 20 zu erzwingen bzw. zu fördern. Ein vom proximalen Ende des Handstücks 2 her fließendes Reinigungsfluid strömt folglich durch die Lagerzwischenräume um die Käfige 28 und Kugeln 30 der Kugellager 20 herum und tritt schließlich am distalen Ende des Handstücks 2 aus. Dabei spült Reinigungsfluid Schmutzpartikel, die sich in den Lagerzwischenräumen angesammelt haben, aus dem Handstück 2 aus.

Zum Einkoppeln der Spülvorrichtung 1 in das Handstück 2 wird die Spülvorrichtung 1 distal in das Handstück 2 manuell eingeführt und solange in dieses eingeschoben, bis der proximale Kopplungsabschnitt 6 der Spülvorrichtung 1 unter Aufwendung einer bestimmten Druckkraft in den Kopplungsabschnitt des Handstücks 2 gebracht ist, also die Kupplungskugeln 36 in die Kopplungstaille 18 greifen. Zum Entkoppeln der Spülvorrichtung 1 aus dem Handstück 2 wird unter Verwendung einer (nicht dargestellten) Entriegelungsfunktion des Handstücks 2 der proximale Kopplungsabschnitt 6 der Spülvorrichtung 1 aus dem Kopplungsabschnitt des Handstücks 2 wieder entfernt, also die Kopplungstaille 18 aus dem Eingriff der Kopplungskugeln 36 gezogen.

In Fig. 4 ist eine perspektivische Seitenansicht eines chirurgischen Instruments 4 mit eingekoppelter Spülvorrichtung 1 gemäß der ersten Ausführungsform der Erfindung dargestellt. Zu sehen ist, dass der distale Abschnitt 8 der Spülvorrichtung 1 im eingekoppelten Zustand aus dem Handstück 2 des chirurgischen Instruments herausragt, um an diesem zum Entkoppeln wieder herausgezogen zu werden.

Fig. 5 zeigt eine perspektivische Seitenansicht einer Spülvorrichtung 1 gemäß einer zweiten Ausführungsform der Erfindung. Die Spülvorrichtung 1 weist einen distalen Abschnitt 8, einen proximalen Kopplungsabschnitt 6 und einen intermediären (teil)zylindrischen Abschnitt 10 auf. Am distalen Abschnitt 8 (im Bereich seines distalen Endes) sind Schaufeln 38 angeordnet, die einen Schaufelkranz bzw. ein Turbinenrad 40 bilden. Die Schaufeln 38 sind in diesem Ausführungsbeispiel integral stoffeinstückig mit der Spülvorrichtung 1, können aber selbstverständlich auch gefügt sein. Der Übergang vom distalen Abschnitt 8 zum intermediären Abschnitt 10, der gegenüber dem distalen Abschnitt 8 umlaufend radial vorspringt, ist gerundet. An den intermediären Abschnitt 10 schließt sich der proximale Kopplungsabschnitt 6 an, welcher gegenüber dem intermediären Abschnitt 10 umlaufend radial zurückversetzt ist. Der Übergang vom intermediären Abschnitt 10 und dem proximalen Kopplungsabschnitt 6 kann gerundet oder in Form einer schrägen Abgleitfläche ausgeführt sein. Der distale Abschnitt 8, der intermediäre Abschnitt 10 und der proximale Kopplungsabschnitt 6 sind jeweils (im Wesentlichen) zylindrisch mit kreisförmigem Querschnitt geformt und koaxial zueinander angeordnet. Dabei weist der proximale Kopplungsabschnitt 6 einen breiteren Querschnitt als der distale Abschnitt 8 auf.

Vom proximalen Ende des proximalen Kopplungsabschnitts 6 bzw. der Spülvorrichtung 1 her ist der proximale Kopplungsabschnitt 6 entlang seiner Längsachse bzw. der Längsachse der Spülvorrichtung 1 durch eine durch den Kopplungsabschnitt 6 hindurchgehende Ausnehmung 42 in drei Klemmarme 44 unterteilt. Die Klemmarme 44 weisen jeweils (im Bereich ihres proximalen Endes) einen Rastabschnitt 46 auf, der eine sich im Wesentlichen senkrecht zur zylindrischen Außenfläche der Kopplungsarme 44 erhebende Rastfläche 48 und eine Abgleitfläche 50 aufweist. Die Abgleitflächen 50 sind jeweils teilkugelförmig gebildet und erstrecken sich auf das proximale Ende Kopplungsabschnitts 6 bzw. der Spülvorrichtung 1. Folglich stellen die proximalen Enden der Abgleitflächen 50 selbst das proximale Ende des Kopplungsabschnitts 6 bzw. der Spülvorrichtung 1 dar. Der Übergang von der zylindrischen Außenfläche des Klemmarms 44 und der Rastfläche 48 kann gerundet oder in Form einer schrägen Abgleitfläche gebildet sein.

In Fig. 6 ist eine perspektivische Frontalansicht der in Fig. 5 gezeigten Spülvorrichtung 1 dargestellt. Zu sehen sind die drei Klemmarme 44, in die der proximale Kopplungsabschnitts 6 durch die Ausnehmung 42 (und entlang dieser) unterteilt ist. Die Ausnehmung 42 durchzieht den proximalen Kopplungsabschnitt 6 von seinem proximalen Ende her entlang seiner Längsrichtung und ausgehend von seinem Querschnittsmittelpunkt radial zu seiner Umfangsaußenseite hin, um eine im Wesentlichen Y-förmige, in Längsrichtung des Kopplungsabschnitts 6 sich erstreckende Durchgangsöffnung zu bilden. Dabei verbreitert sich die Ausnehmung 42 in Richtung ihrer Längserstreckung auf das proximale Ende des Kopplungsabschnitts 6 hin, wobei die Klemmarme 44 über ihre Längserstreckung bis zu den Rastabschnitten 46 einen (im Wesentlichen) konstanten Außendurchmesser aufweisen. Der Spielraum, in dem sich die Klemmarme 44 elastisch radial nach innen verformen können, nimmt also zum proximalen Ende des Kopplungsabschnitts 6 hin zu.

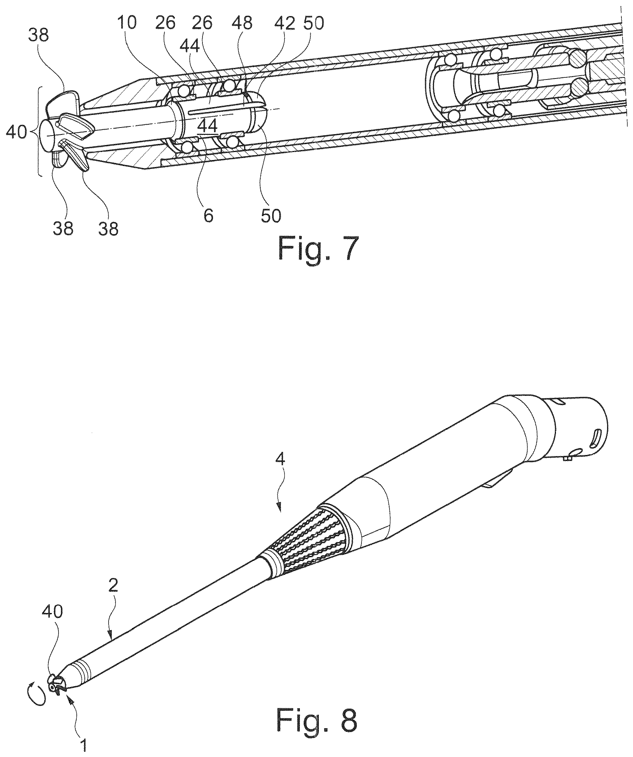

Fig. 7 zeigt eine perspektivische Längsschnittansicht des Handstücks 2 des chirurgischen Instruments 4 mit eingekoppelter Spülvorrichtung 1 gemäß der in den Figuren 5 und 6 dargestellten zweiten Ausführungsform der Erfindung in einer perspektivischen Seitenansicht. Das gezeigte Handstück 2 ist baugleich mit dem in Fig. 2 gezeigten Handstück 2 und wird deshalb nicht erneut beschrieben. Zu sehen ist, dass der proximale Kopplungsabschnitt 6 der Spülvorrichtung 1 an den Innenringen 26 der distalen Kugellager 20 anliegt und den Strömungsweg für ein Reinigungsfluid durch die Durchgangsöffnungen der Innenringe 26 verschließt. Die Klemmarme 44 sind in der eingekoppelten Position der Spülvorrichtung 1 im Handstück 2 (leicht) gegen die Innenringe 26 vorgespannt und bilden mit diesen einen Reib-/Kraftschluss, sodass die Innenringe 26 und die Spülvorrichtung 1 drehfest aneinander gehalten sind.

Ein Reinigungsfluid, das vom proximalen Ende des Handstücks 2 her fließt, strömt also durch den Hohlkörper des Handstücks 2 bis zu den distalen Kugellagern 20 und wird durch den Verschluss der Innenringe 26 durch die Zwischenräume der Kugellager 20 geleitet. Dabei umströmt das Reinigungsfluid die Käfige 28 und Kugeln 30 zwischen den Außenringen 24 und Innenringen 26 der Kugellager 20 und tritt distal vom distaleren Kugellager 20 in einen Strömungskanal, der zwischen der Innenfläche des Hohlkörpers und der Außenfläche des distalen Abschnitts 8 der Spülvorrichtung 1 verläuft und von diesen gebildet wird, um schließlich auf die Schaufeln 38 bzw. das Turbinenrad 40 am distalen Abschnitt 8 der Spülvorrichtung 1 zu treffen und die Spülvorrichtung 1 durch Drehen des Turbinenrads 40 zum Rotieren um ihre Längsachse zu bringen. Durch den Reibschluss zwischen den Klemmarmen 44 und den Innenringen 26 bzw. deren Innenumfangsflächen rotieren diese ebenfalls mit und bringen folglich auch die Kugeln 30 und Käfige 28 in Bewegung. Die Bewegung der Lagerteile gegeneinander und die gleichzeitige Durchströmung mit Reinigungsfluid bewirkt eine verbesserte Schmutzpartikelablösung von den Lagerteilen, sodass die Kugellager 20 noch besser gereinigt werden können.

Zum Einkoppeln der Spülvorrichtung 1 gemäß dieser zweiten Ausführungsform mit dem Handstück 2 wird die Spülvorrichtung 1 distal in das Handstück 2 manuell eingeführt und unter Aufwendung einer bestimmten Druckkraft soweit in das Handstück 2 eingeschoben, bis der proximale Kopplungsabschnitt 6 der Spülvorrichtung 1 sich im Kopplungsabschnitt des Handstücks (hier die Innenumfangsflächen der Innenringe 26) befindet. Beim Einschieben der Spülvorrichtung 1 durch die Innenringe 26 an den distalen Kugellagern 20 des Handstücks 2 gleiten die Abgleitflächen 50 der Rastabschnitte 46 an den distalen Enden der Innenringe 26 entlang und drücken die Klemmarme 44 entlang der Ausnehmung 42 elastisch radial nach innen, bis die Rastabschnitte 46 die Innenringe 26 der distalen Kugellager 20 passiert haben und die Klemmarme 44 sich durch die Vorspannung der elastischen Verformung wieder radial nach außen zurück drücken und die Rastflächen 48 der Rastabschnitte 46 den Innenring 26 des proximaleren Kugellagers 20 hintergreifen, sodass die Spülvorrichtung 1 am proximalen Kopplungsabschnitt 6 nach Art einer zylindrischen Schnappbefestigung axial im Handstück 2 gesichert ist. Dabei bildet der intermediäre Abschnitt 10, der einen breiteren Querschnitt als der proximale Kopplungsabschnitt 6 hat, einen Anschlag in Einschubrichtung der Spülvorrichtung 1 in das Handstück 2.

Zum Entkoppeln der Spülvorrichtung 1 aus dem Handstück 2 wird durch Aufwendung einer bestimmten Zugkraft an der Spülvorrichtung 1 der Schnapp- bzw. Rasteingriff der Rastabschnitte 46 mit den Innenringen 26 und der Reibschluss der Klemmarme 44 mit den Innenumfangsflächen der Innenringe 26 überwunden und die Klemmarme 44 gleiten entlang der gerundeten oder schrägen Abgleitflächen an den Übergängen zu den Rastabschnitten 46 an den proximalen Enden der Innenringe 26 ab, wobei die Klemmarme 44 elastisch radial nach innen gedrückt werden und die Spülvorrichtung 1 aus dem Handstück 2 herausgezogen werden kann.

In Fig. 8 ist eine perspektivische Seitenansicht des chirurgischen Instruments 4 mit eingekoppelter Spülvorrichtung 1 gemäß der zweiten Ausführungsform der Erfindung gezeigt. Zu sehen, ist dass der Schaufelkranz bzw. das Turbinenrad 40 im eingekoppelten Zustand der Spülvorrichtung 1 im Handstück 2 distal aus dem Handstück 2 herausragt und an diesem zum Entkoppeln der Spülvorrichtung 1 von dem Handstück 2 aus dessen Hohlkörper wieder herausgezogen werden kann.

### Bezugszeichenliste

- 1: Spülvorrichtung
- 2: Handstück
- 3: Spüleinsatz
- 4: Chirurgisches Instrument
- 6: Proximaler Kopplungsabschnitt
- 8: Distaler Abschnitt
- 10: Intermediärer Abschnitt
- 12: Stufe
- 14: Außenanlageflächen
- 16: Seitenflächen
- 18: Kopplungstaille
- 20: Distale Kugellager
- 22: Proximale Kugellager
- 24: Außenring
- 26: Innenring
- 28: Käfig
- 30: Kugeln
- 32: Führungshülse
- 34: Innenanlageflächen
- 36: Kopplungskugeln
- 38: Schaufeln
- 40: Turbinenrad
- 42: Ausnehmung
- 44: Klemmarme
- 46: Rastabschnitt
- 48: Rastfläche
- 50: Abgleitfläche

## Patentansprüche

1. Spülvorrichtung (1) zum Durchspülen eines Handstücks (2) eines chirurgischen Instruments (4) mit oder aus einem schaftartigen Spüleinsatz (3), der dafür vorgesehen und ausgelegt ist, um distal in eine am Handstück (2) ausgebildete Werkzeugaufnahme eingesetzt zu werden, und wobei der Spüleinsatz (3) einen proximalen Kopplungsabschnitt (6) zum Koppeln des Spülwerkzeugs (1) mit dem Handstück (2), einen distalen Abschnitt (8) und einen intermediären zylindrischen Abschnitt (10) hat, welcher einen größeren Außenradius als der distale Abschnitt (8) aufweist, wodurch sich an einem Übergang vom distalen Abschnitt (8) zum intermediären Abschnitt (10) ein umlaufender Schaftabsatz oder eine radiale Stufe (12) ausbildet,
**dadurch gekennzeichnet, dass**
der proximale Kopplungsabschnitt (6) am proximalen Ende des Spüleinsatzes (3) vorgesehen und ausgelegt ist, um in den in der Werkzeugaufnahme vorgesehenen Kopplungsabschnitt einzugreifen oder einen Lagerring des Kugellagers (20) in der Werkzeugaufnahme zu hintergreifen, so dass eine axiale Sicherung der Spülvorrichtung (1) in der Werkzeugaufnahme des Handstücks (2) erfolgt.

2. Spülvorrichtung (1) nach Anspruch 1, wobei der Übergang vom distalen Abschnitt (8) zum intermediären Abschnitt (10) gerundet ist.

3. Spülvorrichtung (1) nach Anspruch 1 oder 2, wobei die Spülvorrichtung (1) integral stoffeinstückig gebildet ist.

4. Spülvorrichtung (1) nach einem der Ansprüche 1 bis 3, wobei der distale Abschnitt (8) ein Turbinenrad (40) aufweist.

5. Spülvorrichtung (1) nach einem der Ansprüche 1 bis 4, wobei der proximale Kopplungsabschnitt (6) mindestens zwei elastisch, vorzugsweise federelastisch, deformierbare und/oder sich in Längsrichtung der Spülvorrichtung (1) erstreckende, Klemmarme (44), insbesondere Spreizarme, aufweist.

6. Spülvorrichtung (1) nach Anspruch 5, wobei die Klemmarme (44) jeweils einen radial vorspringenden Rastabschnitt (46) mit einer Abgleitfläche (50) aufweisen, die sich auf das proximale Ende des Klemmarms (44) hin erstreckt.

7. Spülvorrichtung (1) nach einem der Ansprüche 1 bis 6, wobei der intermediäre Abschnitt (10) ringförmig und/oder hülsenförmig ist.

8. Spülvorrichtung (1) nach einem der Ansprüche 1 bis 7, wobei die Spülvorrichtung (1), vorzugsweise die gesamte Spülvorrichtung (1), zumindest aber der distale Abschnitt (8) und der intermediäre Abschnitt (10), einen Vollzylinder aufweist oder ein Vollzylinder ist.

9. Spülsystem zum Durchspülen eines Handstücks (2) eines chirurgischen Instruments (4) mit einer Spülvorrichtung (1) zum Durchspülen des Handstücks (2) des chirurgischen Instruments (4) mit oder aus einem schaftartigen Spüleinsatz (3), der dafür vorgesehen und ausgelegt ist, um distal in eine am Handstück (2) ausgebildete Werkzeugaufnahme eingesetzt zu werden, und wobei der Spüleinsatz (3) einen proximalen Kopplungsabschnitt (6) zum Koppeln des Spülwerkzeugs (1) mit dem Handstück (2), einen distalen Abschnitt (8) und einen intermediären zylindrischen Abschnitt (10) hat, welcher einen größeren Außenradius als der distale Abschnitt (8) aufweist, wodurch sich an einem Übergang vom distalen Abschnitt (8) zum intermediären Abschnitt (10) ein umlaufender Schaftabsatz oder eine radiale Stufe (12) ausbildet, und dem Handstück (2) des chirurgischen Instruments (4), das die Werkzeugaufnahme in Form eines Hohlkörpers ausbildet, in den die Spülvorrichtung (1) distal einführbar ist,
**dadurch gekennzeichnet, dass**
die Werkzeugaufnahme weiter einen Kopplungsabschnitt zum Koppeln der Spülvorrichtung (1) aufweist und in einem distalen Abschnitt der Werkzeugaufnahme mindestens ein Kugellager (20) vorgesehen ist, das einen eine Durchgangöffnung bildenden Innenring (26) aufweist, die Spülvorrichtung (1) in gekoppeltem Zustand des Spülsystems einen Strömungsweg durch das Handstück (2) entlang einer Innenumfangsfläche des Innenrings (26) verschließt und einen Strömungsweg distal von dem zumindest einen Kugellager (20) wieder freigibt.

10. Spülsystem nach Anspruch 9, wobei der Kopplungsabschnitt der Werkzeugaufnahme dazu angepasst ist, mit dem Kopplungsabschnitt der Spülvorrichtung (1) eine werkzeuglose, form- und/oder reibschlüssige Kopplung zu bilden.

11. Spülsystem nach Anspruch 9 oder 10, wobei die Spülvorrichtung (1) drehfest an der Innenumfangsfläche des Innenrings (26) anliegt und/oder drehfest mit dem Innenring (26) verbunden ist.

12. Spülsystem nach einem der Ansprüche 9 bis 11, wobei der Kopplungsabschnitt der Werkzeugaufnahme entlang der Innenumgangsfläche des Innenrings (26) verläuft und dazu angepasst ist, mit dem proximalen Kopplungsabschnitt (6) der Spülvorrichtung (1) eine Klemmkupplung zu bilden.

13. Spülsystem nach Anspruch 12, wobei die Spülvorrichtung (1) eine Spülvorrichtung (1) nach einem der Ansprüche 5 bis 8 ist und die Klemmarme (44) mit dem Innenring (26) eine zylindrische Schnappbefestigung bilden.

## Claims

1. A flushing device (1) for flushing a handpiece (2) of a surgical instrument (4) with or from a shaft-like flushing insert (3), which is provided and configured to be inserted distally into a tool receptacle formed on the handpiece (2), and wherein the flushing insert (3) has a proximal coupling portion (6) for coupling the flushing tool (1) to the handpiece (2), a distal portion (8) and an intermediate cylindrical portion (10), which has a larger outer radius than the distal portion (8), whereby a circumferential shaft shoulder or a radial step (12) is formed at a transition from the distal portion (8) to the intermediate portion (10),
**characterized in that**
the proximal coupling portion (6) is provided and configured at the proximal end of the flushing insert (3) to engage in the coupling portion provided in the tool receptacle or to engage behind a bearing ring of the ball bearing (20) in the tool receptacle, so that an axial securing of the flushing device (1) in the tool receptacle of the handpiece (2) takes place.

2. The flushing device (1) according to claim 1, wherein the transition from the distal portion (8) to the intermediate portion (10) is rounded.

3. The flushing device (1) according to claim 1 or 2, wherein the flushing device (1) is integrally formed in one piece.

4. The flushing device (1) according to one of claims 1 to 3, wherein the distal portion (8) has a turbine wheel (40).

5. The flushing device (1) according to one of claims 1 to 4, wherein the proximal coupling portion (6) has at least two clamping arms (44), in particular spreading arms, which are elastically, preferably spring-elastically, deformable and/or extend in the longitudinal direction of the flushing device (1).

6. The flushing device (1) according to claim 5, wherein the clamping arms (44) each have a radially projecting latching portion (46) with a sliding surface (50), which extends towards the proximal end of the clamping arm (44).

7. The flushing device (1) according to one of claims 1 to 6, wherein the intermediate portion (10) is annular and/or sleeve-shaped.

8. The flushing device (1) according to one of claims 1 to 7, wherein the flushing device (1), preferably the entire flushing device (1), but at least the distal portion (8) and the intermediate portion (10), has a solid cylinder or is a solid cylinder.

9. A flushing system for flushing a handpiece (2) of a surgical instrument (4) with a flushing device (1) for flushing the handpiece (2) of the surgical instrument (4) with or from a shaft-like flushing insert (3), which is provided and configured to be inserted distally into a tool receptacle formed on the handpiece (2), and wherein the flushing insert (3) has a proximal coupling portion (6) for coupling the flushing tool (1) to the handpiece (2), a distal portion (8) and an intermediate cylindrical portion (10), which has a larger outer radius than the distal portion (8), whereby a circumferential shaft shoulder or a radial step (12) is formed at a transition from the distal portion (8) to the intermediate portion (10), and the handpiece (2) of the surgical instrument (4), which forms the tool receptacle in the form of a hollow body, into which the flushing device (1) can be inserted distally,
**characterized in that** the tool receptacle further has a coupling portion for coupling the flushing device (1) and at least one ball bearing (20) is provided in a distal portion of the tool receptacle, which has an inner ring (26) forming a passage opening, the flushing device (1) in the coupled state of the flushing system closes a flow path through the handpiece (2) along an inner circumferential surface of the inner ring (26) and releases a flow path distally from the at least one ball bearing (20) again.

10. The flushing system according to claim 9, wherein the coupling portion of the tool receptacle is adapted to form a tool-free, form-fitting and/or friction-fitting coupling with the coupling portion of the flushing device (1).

11. The flushing system according to claim 9 or 10, wherein the flushing device (1) bears in a rotationally fixed manner against the inner circumferential surface of the inner ring (26) and/or is connected in a rotationally fixed manner to the inner ring (26).

12. The flushing system according to one of claims 9 to 11, wherein the coupling portion of the tool receptacle runs along the inner circumferential surface of the inner ring (26) and is adapted to form a clamping coupling with the proximal coupling portion (6) of the flushing device (1).

13. The flushing system according to claim 12, wherein the flushing device (1) is a flushing device (1) according to one of claims 5 to 8 and the clamping arms (44) form a cylindrical snap fastening with the inner ring (26).

## Revendications

1. Dispositif de rinçage (1) destiné au rinçage d'une pince à main (2) d'un instrument chirurgical (4) avec ou à partir d'un embout de rinçage en forme de tige (3) qui est prévu et conçu pour être inséré de manière distale dans un porte-outil formée au niveau de la pièce à main (2), et dans lequel l'embout de rinçage (3) a une section de couplage proximale (6) destinée au couplage de l'outil de rinçage (1) avec la pièce à main (2), une section distale (8) et une section cylindrique intermédiaire (10), qui présente un rayon externe plus grand que la section distale (8), selon lequel se forme au niveau d'une transition de la section distale (8) à la section intermédiaire (10) un talon droit enveloppant ou un épaulement radial (12),
**caractérisé en ce que**
la section de couplage proximale (6) est prévue et conçue au niveau de l'extrémité proximale de l'embout de rinçage (3) pour s'encliqueter dans la section de couplage prévue dans le porte-outil ou pour saisir par l'arrière une bague de roulement du roulement à billes (20) dans le porte-outil, de sorte qu'un blocage axial du dispositif de rinçage (1) dans le porte-outil de la pièce à main (2) se produit.

2. Dispositif de rinçage (1) selon la revendication 1, dans lequel la transition de la section distale (8) à la section intermédiaire (10) est arrondie.

3. Dispositif de rinçage (1) selon la revendication 1 ou 2, dans lequel le dispositif de rinçage (1) est formé intégralement d'une seule pièce de tissu.

4. Dispositif de rinçage (1) selon l'une quelconque des revendications 1 à 3, dans lequel la section distale (8) présente une roue de turbine (40).

5. Dispositif de rinçage (1) selon l'une quelconque des revendications 1 à 4, dans lequel la section de couplage proximale (6) présente au moins deux bras de serrage (44), en particulier des bras d'écartement, de préférence élastiques, déformables et/ou s'étendant dans la direction longitudinale du dispositif de rinçage (1).

6. Dispositif de rinçage (1) selon la revendication 5, dans lequel les bras de serrage (44) présentent respectivement une section d'enclenchement (46) avec une surface de glissement (50) qui s'étend vers l'extrémité proximale du bras de serrage (44).

7. Dispositif de rinçage (1) selon l'une quelconque des revendications 1 à 6, dans lequel la section intermédiaire (10) est de forme annulaire et/ou en forme de manchon.

8. Dispositif de rinçage (1) selon l'une quelconque des revendications 1 à 7, dans lequel le dispositif de rinçage (1), de préférence l'ensemble du dispositif de rinçage (1), mais au moins la section distale (8) et la section intermédiaire (10), présente un cylindre plein ou est un cylindre plein.

9. Dispositif de serrage destiné au rinçage d'une pince à main (2) d'un instrument chirurgical (4) avec un dispositif de rinçage (1) destiné au rinçage de la pièce à main (2) de l'instrument chirurgical (4) avec ou à partir d'un embout de rinçage en forme de tige (3) qui est prévu et conçu pour être inséré de manière distale dans le porte-outil formé au niveau de la pièce à main (2), et dans lequel l'embout de rinçage (3) a une section de couplage proximale (6) destinée au couplage de l'outil de rinçage (1) avec la pièce à main (2), une section distale (8) et une section cylindrique intermédiaire (10), qui présente un rayon externe plus grand que la section distale (8), selon lequel se forme au niveau d'une transition de la section distale (8) à la section intermédiaire (10) un talon droit enveloppant ou un épaulement radial (12) et à la pièce à main (2) de l'instrument chirurgical (4) qui forme le porte-outil en forme de corps creux dans lequel le dispositif de rinçage (1) peut être introduit de manière distale,
**caractérisé en ce que**
le porte-outil présente en outre une section de couplage destinée au couplage du dispositif de rinçage (1) et au moins un roulement à billes (20) est prévu dans une section distale du porte-outil, lequel roulement à billes présente une bague interne (26) formant une ouverture de passage , le dispositif de rinçage (1) verrouille un chemin d'écoulement à travers la pièce à main (2) le long d'une surface périphétique interne de la bague interne (26) dans l'état couplé du système de rinçage et libère à nouveau un chemin d'écoulement de manière distale à partir de l'au moins un roulement à billes (20).

10. Système de rinçage selon la revendication 9, dans lequel la section de couplage du porte-outil est adaptée pour former avec la section de couplage du dispositif de rinçage (1) un couplage sans outil, par complémentarité de formes et/ou par friction.

11. Système de rinçage selon la revendication 9 ou 10, dans lequel le dispositif de rinçage (1) repose de manière fixe en rotation au niveau de la surface périphérique interne de la bague interne (26) et/ou est relié de manière fixe en rotation avec la bague interne (26).

12. Système de rinçage selon l'une quelconque des revendications 9 à 11, dans lequel la section de couplage du porte-outil s'étend le long de la surface périphérique interne de la bague interne (26) et est adaptée pour former avec la section de couplage proximale (6) du dispositif de rinçage (1) un couplage à serrage.

13. Système de rinçage selon la revendication 12, dans lequel le dispositif de rinçage (1) est un dispositif de rinçage (1) selon l'une quelconque des revendications 5 à 8 et les bras de serrage (44) forment avec la bague interne (26) une fixation par encliquetage cylindrique.
